# EUROPEAN PATENT SPECIFICATION

(11) **EP 1 490 401 B1**
(45) Date of publication and mention of the grant of the patent: **25.04.2007**
(21) Application number: 03745335.4
(22) Date of filing: 28.03.2003
(51) Int. Cl.: C07K 14/475, A61K 38/18, A61K 38/12

(54) **VEGF PEPTIDES AND THEIR USE**
VEGF-PEPTIDE UND DEREN VERWENDUNG
PEPTIDES VEGF ET LEUR UTILISATION

(30) Priority: 02.04.2002 GB 0207644
(43) Date of publication of application: 29.12.2004
(73) Proprietor: Ark Therapeutics Limited, London W1W 6XB (GB)
(72) Inventor: Selwood, D., Wolfson Inst. for Biomedical Research, London WC1E 6AU (GB); Löhr, M., Wolfson Inst. for Biomedical Research, London WC1E 6AU (GB); Zachary, I., Wolfson Inst. for Biomedical Research, London WC1E 6AU (GB)
(74) Representative: Perry, Robert Edward
(86) International application number: PCT/GB2003/001375
(87) International publication number: WO 2003/082918

(56) References cited:
- WO-A-93/08473
- WO-A-99/29861
- HAIYAN JIA ET AL: "PEPTIDES ENCODED BY EXON 6 OF VEGF INHIBIT ENDOTHELIAL CELL BIOLOGICAL RESPONSES AND ANGIOGENESIS INDUCED BY VEGF" BIOCHEMICAL AND BIOPHYSICAL RESEARCH COMMUNICATIONS, ACADEMIC PRESS INC. ORLANDO, FL, US, vol. 283, no. 1, 2001, pages 164-173, XP002941058 ISSN: 0006-291X
- SOKER S ET AL: "Characterization of novel Vascular Endothelial Growth Factor (VEGF) Receptors on tumor cells that bind VEGF165 via its exon 7-encoded domain" JOURNAL OF BIOLOGICAL CHEMISTRY, AMERICAN SOCIETY OF BIOLOGICAL CHEMISTS, BALTIMORE, MD, US, vol. 271, no. 10, 8 March 1996 (1996-03-08), pages 5761-5767, XP002097905 ISSN: 0021-9258 cited in the application
- SOKER S ET AL: "Inhibition of Vascular Endothelial Growth Factor (VEGF)-induced endothelial cell proliferation by a peptide corresponding to the exon7-encoded domain of VEGF165" JOURNAL OF BIOLOGICAL CHEMISTRY, AMERICAN SOCIETY OF BIOLOGICAL CHEMISTS, BALTIMORE, MD, US, vol. 272, no. 50, 12 December 1997 (1997-12-12), pages 31582-31588, XP002098056 ISSN: 0021-9258 cited in the application
- BAINBRIDGE JAMES W B ET AL: "A peptide encoded by exon 6 of VEGF (EG3306) inhibits VEGF-induced angiogenesis in vitro and ischaemic retinal neovascularisation in vivo." BIOCHEMICAL AND BIOPHYSICAL RESEARCH COMMUNICATIONS, vol. 302, no. 4, 21 March 2003 (2003-03-21), pages 793-799, XP002245130 ISSN: 0006-291X

### SEQUENCE LISTING

<110> Ark Therapeutics Ltd.
<120> VEGF Peptides and Their Use
<130> REP06595WO
<140> (not yet known)
   < 141 > 2003-03-28
<150> 0207644.6
   < 151 > 2002-04-02
<160> 2
<170> Patentln Ver. 2.1
<210> 1
   <211> 24
   <212> PRT
   <213> Artificial Sequence
<220>
   <223> Description of Artificial Sequence: Peptide
<400> 1
   Cys Ser Cys Lys Asn Thr Asp Ser Arg Cys Lys Ala Arg Gln Leu Glu
   1               5                   10                  15
   Leu Asn Glu Arg Thr Cys Arg Cys
               20
<210> 2
   <211> 28
   <212> PRT
   <213> Artificial Sequence
<220>
   <223> Description of Artificial Sequence: Peptide
<400> 2
   Ser Cys Lys Asn Thr Asp Ser Arg Cys Lys Ala Arg Gln Leu Glu Leu
   1 5 10 15
   Asn Glu Arg Thr Cys Arg Cys Asp Lys Pro Arg Arg
   20 25

## Description

### Field of the Invention

This invention relates to peptides which are fragments of VEGF (vascular endothelial growth factor) and which have activity of potential benefit in therapy.

### Background of the Invention

VEGF-A is a secreted polypeptide which is essential for formation of the vascular system in embryogenesis and plays a major role in angiogenesis in a variety of disease states. VEGF expression is upregulated by hypoxia and several cytokines in diverse cell types, and elicits multiple biological activities *in vivo* and *in vitro,* including the differentiation, proliferation, migration and survival of endothelial cells, increased vascular permeability, monocyte migration, and increased endothelial production of the vasodilatory factors NO and prostacyclin.

Human VEGF-A exists in multiple isoforms, of 121, 145, 165, 189 and 206 amino acids, generated by alternative mRNA splicing, of which VEGF₁₂₁, VEGF₁₄₅ and VEGF₁₆₅ are known to be secreted and biologically active. Two distinct protein tyrosine kinase receptors forVEGF have been identified, i.e. Flt-1 (VEGFR1) and KDR/Flk-1 (VEGFR2). KDR/flk-1 is thought to be the receptor which primarily mediates the mitogenic effects of VEGF in endothelial cells and angiogenesis *in vivo;* the function of Flt-1 in endothelial cells is unknown. A non-tyrosine kinase transmembrane protein, neuropilin-1 (NP-1), has been identified as an additional receptor for VEGF, which specifically binds VEGF₁₆₅. VEGF promotes the survival of tumour cells expressing NP-1, including some breast carcinoma cells (Bachelder *et al*, Cancer Res 61: 5736-5740, 2001). The role of NP-1 in mediating biological effects of VEGF is still largely unknown.

NP-1 is a receptor for a family of molecules called semaphorins or collapsins which play a key role in the guidance of neuronal axons during mammalian development. In particular, NP-1 is known to mediate the growth cone-collapsing and chemorepulsive activity of semaphorin 3.

Soker *et al*, J. Biol. Chem. 271(10): 5761-5767 (1996), discloses that a GSTfusion protein containing the 44 amino acids encoded by VEGF exon 7 bind to NP-1.

Soker *et al*, J. Biol. Chem. 272(10): 31582-31588 (1997), discloses that a region of exon 7 is necessary for inhibition of VEGF binding to HUVECs. The shortest active peptide (SEQ ID NO. 1) is

CSCKNTDSRCKARQLELNERTCRC

i.e. VEGF (137-160), or amino acids 22-44 of exon 7 and amino acid 1 of exon 8. The terminal cysteine residue (C¹³⁷ in VEGF) is apparently essential for activity and the molecule's 3D structure. It is suggested that there may be intradisulfide bonding within the VEGF monomer.

### Summary of the Invention

Surprisingly, it has been found that certain peptides have NP-1 antagonist activity. A novel peptide according to the present invention, has SEQ ID NO. 2, i.e. the amino acid sequence

SCKNTDSRCKARQLELNERTCRCDKPRR

or a fragment thereof that substantially retains NP-1 antagonist activity, in cyclic form.

The given sequence corresponds to amino acids 138 to 165 within VEGF, i.e. including part at least of exon 8. Surprisingly, activity has been found in peptides lacking the Cys residue indicated by Soker *et al* to be essential.

The invention also encompasses variants of the given sequence, in which the novel activity, i.e. the NP-1 antagonism, is retained without unexpected structural variation. Thus, the given sequence may include isosteric or homologous replacements or derivatisations that render the peptide relatively stable.

### Description of Preferred Embodiments

Peptides of the invention may be synthesised by known methods. Examples are given below. In particular, a linear peptide may be produced by automated peptide synthesis, followed by cyclisation. Known cyclisation procedures include those described by Tam *et al*, JACS 113:6657-62 (1991). Other cyclisations, e.g. Mitsunobu or olefin metathesis ring closure, may also be used.

A peptide of this invention preferably has 4 Cys residues. It is preferably bicyclic.

As indicated above, peptides of the invention may include modifications of the given sequence. Such modifications are well known to those skilled in the art. Isosteric replacements include Abu for Cys (this may be desirable where the peptide should retain an even number of Cys residues for cyclisation), Phe for Tyr and different alkyl/aryl substituents. The shifting of substituents within an amino acid residue, from a C atom to a N atom, to produce peptoids having greater resistance to proteolysis, and other modifications, are known and are included within the scope of this invention. A specific peptide reported here is N-acetylated; otherterminal modifications will also be known to those of ordinary skill in the art. Such modified peptides may act as prodrugs, and/or have modified immunogenicity.

The NP-1 antagonist properties of a peptide of this invention may be determined by the procedure described below. The level of activity is preferably at least 25 or 50% as great as that for the bicyclic 28-mer that has been synthesised.

The activity of peptides of the invention means that they may be useful in the treatment of diseases in which NP-1 may have a significant role in pathology. For therapeutic use, peptides of the invention may be formulated and administered by procedures, and using components, known to those of ordinary skill in the art. The appropriate dosage of the peptide may be chosen by the skilled person having regard to the usual factors such as the condition of the subject to be treated, the potency of the compound, the route of administration etc. Suitable routes of administration include intramuscular, intranasal and subcutaneous.

A NP-1 antagonist may compete with semaphorin 3 for binding to NP-1, and thereby antagonise effects of semaphorin 3 on axonal outgrowth and migration in nerve cells. Potential applications of this are in promoting neurite outgrowth, in stimulating nerve repair or treating neurodegeneration. Further, an NP-1 antagonist may promote the survival of semaphorin 3-responsive neurones, an effect that would confirm or enhance its utility in the applications given above, and may extend these applications, e.g. to treating neuronal death caused by episodes of ischaemia as in stroke and some eye diseases.

NP-1 plays an important role in angiogenesis and may be essential for VEGF-induced angiogenesis in cancer, eye disease, rheumatoid arthritis and other diseases. Therefore, NP-1 antagonists may have applications in the inhibition of VEGF-dependent angiogenesis in disease.

NP-1 antagonists may also play a role in immunosuppression. Therefore, it may be useful to give a peptide of the invention before, during or after a transplant.

In addition, a NP-1 antagonist may compete with VEGF for binding to NP-1 in tumour cells and promote cell death in NP-1-expressing tumour cells. Potential applications of this are in anti-cancer therapy.

The following Examples illustrate the invention.

### Abbreviations

MBHA, methylbenzhydrylamine; Fmoc, 9-fluorenylmethoxy-carbonyl; Ala, alanine; Arg, arginine; Asn, asparagine; Asp, aspartic acid; Abu, aminobutyric acid; Cys, cysteine; Gln, glutamine; Glu, glumatic acid; Gly, glycine; His, histidine; Ile, isoleucine; Leu, leucine; Lys, lysine; Met, methionine; Phe, phenylalanine; Pro, proline; Ser, serine; Thr, threonine; Trp, tryptophan; Tyr, tyrosine; Val, valine; Pbf, 2,2,4,6,7-pentamethyldihydrobenzofuran-5-sulfonyl; Pmc, 2,2,5,7,8-pentamethylchroman-6-sulfonyl; Trt, trityl; tBu, *tert*-butyl; Boc, butoxycarbonyl; Pybop, benzotriazol-1-yl-oxy-tris-pyrrolidino-phosphonium hexafluorophosphate; NMM, N-methyl morpholine; DCM, dichloromethane; DMF, dimethylformamide; TBME, t-butyl methyl ether; TFA, trifluoroacetic acid; HPLC, high performance liquid chromatography; LC-MS, liquid chromatography mass spectrometry; Å, Angström; AAA, amino acid analysis; DMSO, dimethyl sulfoxide; VEGF, vascular endothelial growth factor.

### Small Scale Peptide Syntheses

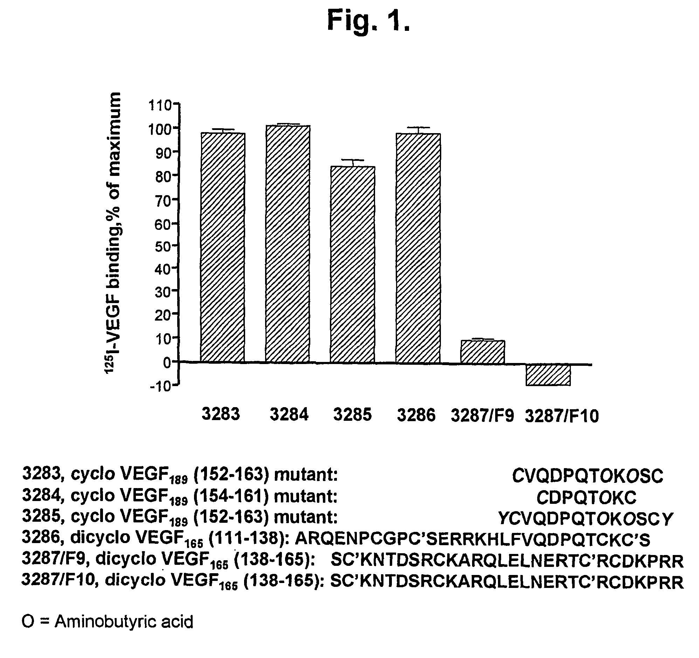

The peptides shown in Fig. 1 were synthesised on an automated AMS 422 Multiple Peptide Synthesiser using the solid phase approach. The Rink Amide MBHA resin (0.59 and 0.68 mmol/g loading) and the N-Fmoc strategy with orthogonal protection (Acm, t-Bu) of the Cys side chains of derivatives to be cyclised were applied. The desired peptide was synthesised on a 25 µM scale and coupled once with a basis coupling time of 30 minutes. The resin and the amino acid derivatives, Fmoc-Ala-OH.H₂O, Fmoc-Arg(Pbf/Pmc)-OH, Fmoc-Asn(Trt)-OH, Fmoc-Asp(OtBu)-OH, Fmoc-Cys(Trt)-OH, Fmoc-Gln(Trt)-OH, Fmoc-Glu(OtBu)-OH, Fmoc-Gly-OH, Fmoc-His(Trt)-OH, Fmoc-Ile-OH, Fmoc-Leu-OH, Fmoc-Lys(Boc)-OH, Fmoc-Met-OH, Fmoc-Phe-OH, Fmoc-Pro.H₂O, Fmoc-Ser(tBu)-OH, Fmoc-Thr(tBu)-OH, Fmoc-Trp(Boc)-OH, Fmoc-Tyr(tBu)-OH and Fmoc-Val-OH were purchased from Calbiochem-Novabiochem UK Ltd. (Nottingham, UK) or Alexis (Nottingham, UK).

Each amino acid was sequentially coupled to the growing peptide chain from the C- to the N-terminus, applying Pybop (Calbiochem-Novabiochem) and NMM (Rathburn Chemicals, Walkerburn, UK) as coupling reagents via the active ester method. Removal of the N-Fmoc protecting group was carried out with 20% piperidine in DMF (Rathburn Chemicals, Walkerburn, Scotland) followed by sequential washes with DMF and DCM. Automatic acetylation was carried out after the synthesis of each peptide with a 4-fold excess of acetic acid (0.7 molar, Rathburn Chemicals, Winterburn Scotland) based on the substitution of the Rink-Amide-MBHA resin. The coupling reagent, Pybop, NMM and all amino acid derivatives were dissolved in DMF (0.7 M, 4-fold excess based on the substitution of the Rink-Amide-MBHA-resin) except for the amino acids Fmoc-His(Trt)-OH and Fmoc-Phe-OH. These protected amino acid derivatives were dissolved in N-methylpyrrolidone. All solvents used were of HPLC-grade quality. The peptides were cleaved from the resin with simultaneous deprotection using 90% TFA at room temperature for 3 hours in the presence of 5% thioanisole, 2.5% water and 2.5% ethanedithiol as a scavenger of reactive cations generated. The cleavage mixture was filtered and precipitated in ice-cold TBME. The remaining resin was washed once with the cleavage reagent, filtered and combined with the previous fractions. The precipitates were collected after centrifugation, washed three times with ice-cold TBME and allowed to dry overnight at room temperature. The crude peptides were dissolved in 15% aqueous acetic acid and lyophilised for 2 days (-40°C, 6 mbar).

### Purification and Characterisation

The crude peptides were analysed by analytical LC-MS on a Quattro LC Mass Spectrometer from Micromass with a Hewlett-Packard HPLC instrument, model 1100 using analytical reverse-phase columns (column Alltech Hypersil PEP reverse-phase column, 100 Å, C₈, 5 µ (250 x 4.6 mm) 0% → 50% acetonitrile in 20 minutes. The separations were monitored at a wavelength of 215 nm for the amide bond absorbance with a flow rate of 1 mL/min. The crude peptides were purified by preparative reverse-phase HPLC (Gilson), monitored at 215 nm and eluted at a flow rate of 20 mL/min. The same mobile phase as stated for the LC-MS analysis of the crude peptides was used. The crude peptides were purified using an Alltech Hypersil PEP reverse-phase column, 100 Å, C₈, 8 µ (250 x 22 mm). They were eluted with 0% → 50% acetonitrile in 20 minutes. The analogues were greater than 95% pure using high performance liquid chromatography (LC-MS) and had the expected amino acid analysis.

Various different gradients were applied for the elution of the peptides which were monitored at 215 nm. The organic phase, acetonitrile, and the aqueous phase both contain 0.1% TFA and 3% 1-propanol. The gradients and flow rates are listed below. The percentage indicates the proportion of the organic phase. 0% → 50% in 20 minutes, flow rate of 1 mL/min.

### Larger Scale Synthesis of Linear SEQ ID NO. 2

SEQ ID NO. 2 was synthesised by an automated single solid-phase approach (Applied Biosystems 433A peptide synthesiser) using the Fmoc-Arg(Pbf)-p-alkoxybenzyl alcohol resin (0.59 mmol/g loading). Amino acids were attached by Fmoc strategy on a 0.25 mmol scale using Fastmoc^{™} chemistry with a single coupling time of 21 minutes. The resin and the amino acid derivatives, Fmoc-Ala-OH, Fmoc-Arg(Pbf)-OH, Fmoc-Asn(Trt)-OH, Fmoc-Asp(OtBu)-OH, Fmoc-Cys(Trt)-OH (positions 9 and 23), Fmoc-Cys(Acm)-OH (positions 2 and 21), Fmoc-Gln(Trt)-OH, Fmoc-Glu(OtBu)-OH, Fmoc-Leu-OH, Fmoc-Lys(Boc)-OH, Fmoc-Pro-OH, Fmoc-Ser(tBu)-OH, Fmoc-Thr(tBu)-OH and Fmoc-Tyr(tBu)-OH, were purchased from Bachem AG, Bubendorf, Switzerland. Each amino acid was added sequentially to the growing peptide chain from the C- to the N-termini applying 0.45M 2-(1H-benzotriazol-1-yl)-1,1,3,3-tetramethyluronium hexafluorophosphate (HBTU)/1-hydroxybenzotriazole (HOBt) in DMF (Applied Biosystems, Warrington, UK) and N,N-diisopropylethylamine (DIEA, Fisher Chemicals, Loughborough, UK) as coupling reagents. Removal of the N-Fmoc protecting group was carried out with 20% piperidine (Romil, Cambridge, UK) in N-methylpyrrolidone (NMP, M56 Chemicals, Runcorn, UK) followed by sequential washes with NMP. The coupling reagent, HBTU/HOBt is 3.6-fold excess (0.9 mmol) and all amino acid derivatives 4-fold excess (1.0 mmol). All solvents used were of HPLC-grade quality.

### Synthesis of Linear Ac-SEQ ID NO. 2

Ac-SEQ ID NO. 2 was synthesized by an automated multiple solid-phase approach (Rainin Symphony multiple peptide synthesiser) using the Fmoc-Arg(Pbf)-p-alkoxybenzyl alcohol resin (0.59 mmol/g loading). Amino acids were attached by Fmoc strategy on a 0.2 mmol scale (peptides 3) using Fmoc chemistry with a single coupling time of 20 minutes. The resin and the amino acid derivatives, Fmoc-Ala-OH, Fmoc-Abu-OH, Fmoc-Arg(Pbf)-OH, Fmoc-Asn(Trt)-OH, Fmoc-Asp(OtBu)-OH, Fmoc-Cys(Trt)-OH (peptide 3 positions 9 and 23), Fmoc-Cys(Acm) (peptide 3 positions 2 and 21), Fmoc-Gln(Trt)-OH, Fmoc-Glu(OtBu)-OH, Fmoc-Leu-OH, Fmoc-Lys(Boc)-OH, Fmoc-Pro-OH, Fmoc-Ser(tBu)-OH, Fmoc-Thr(tBu)-OH and Fmoc-Tyr(tBu)-OH were purchased from Bachem AG, Bubendorf, Switzerland. Each amino acid was added sequentially to the growing peptide chain from the C- to the N- termini applying 2-(1H-benzotriazol-1-yl)-1,1,3,3-tetramethyluronium tetrafluoroborate (TBTU, Severn Biotech Ltd, Kidderminster, UK) and N,N-diisopropylethylamine (DIEA, Fisher Chemicals, Loughborough, UK) as coupling reagents. Removal of the N-Fmoc protecting group was carried out with 20% piperidine (Romil, Cambridge, UK) in N-methylpyrrolidone (NMP, M56 Chemicals, Runcorn, UK) followed by sequential washes with NMP. The coupling reagent, TBTU (4-fold excess), was dissolved in DMF (Apollo Scientific, Stockport UK) and all amino acid derivatives (4-fold excess) and DIEA (8-fold excess) in NMP. Automatic N-terminal acetylation was carried out after synthesis (peptides 3) using an excess of a capping mixture of acetic anhydride/lutidine/DMF/DCM (2:1:9:8). All solvents used were of HPLC-grade quality.

### Synthesis of Linear Ac-SEQ ID NO. 2 - NH₂

Ac-SEQ ID NO. 2 - NH₂ was synthesized using a semi-automated solid-phase approach (Labortec AG SP4000 peptide synthesiser) using the tricyclic amide linker resin (0.63 mmol/g loading). Amino acids were attached by Fmoc strategy on a 4 mmol scale using Fmoc chemistry with a single coupling time of 60 minutes. The completion of each coupling step was monitored using the Kaiser colour test. Recouplings were performed until a negative colour test was obtained. The resin and the amino acid derivatives, Fmoc-Ala-OH, Fmoc-Arg(Pbf)-OH, Fmoc-Asn(Trt)-OH, Fmoc-Asp(OtBu)-OH, Fmoc-Cys(Trt)-OH (positions 9 and 23), Fmoc-Cys(Acm)-OH (positions 2 and 21), Fmoc-Gln(Trt)-OH, Fmoc-Glu(OtBu)-OH, Fmoc-Leu-OH, Fmoc-Lys(Boc)-OH, Fmoc-Pro-OH, Fmoc-Ser(tBu)-OH, Fmoc-Thr(tBu)-OH and Fmoc-Tyr(tBu)-OH were purchased from Bachem AG, Bubendorf, Switzerland. Each amino acid was added sequentially to the growing peptide chain from the C- to the N- termini applying 2-(1H-benzotriazol-1-yl)-1,1,3,3-tetramethyluronium tetrafluoroborate (TBTU, Severn Biotech Ltd, Kidderminster, UK) and N,N-diisopropylethylamine (DIEA, Fisher Chemicals, Loughborough, UK) as coupling reagents. Removal of the N-Fmoc protecting group was carried out with 20% piperidine (Romil, Cambridge, UK) in DMF, followed by sequential washes with DMF and propan-2-ol. The coupling reagent, TBTU, all amino acid derivatives (3-fold excess) and DIEA (6-fold excess) were dissolved in DMF. N-terminal acetylation was carried out after synthesis using an excess of a capping mixture of acetic anhydride/lutidine/DMF/DCM (2:1:9:8). All solvents used were of HPLC-grade quality.

### Peptide cleavage

The peptides were cleaved from the resin with simultaneous deprotection using 82.5% TFA at room temperature for 3 hours in the presence of 5% thioanisole, 5% water, 2.5% ethanedithiol and 6% (w/v) phenol. The cleavage mixture was filtered and precipitated in ice-cold diethyl ether. The remaining resin was washed once with TFA, filtered, and combined with the previous fractions. The precipitates were stored at 4°C overnight and were collected by filtration, washed with ice-cold diethyl ether, and allowed to dry at room temperature. The crude peptides were dissolved in TFA/acetonitrile/water and lyophilized overnight (-50°C, 6 mbar).

### Characterization of crude peptides

Peptides were characterised by reverse-phase HPLC (Gilson) using an analytical C-18 column (Vydac 218TP54, 250 x 4.6mm, 5 µm particle size, and 300 Å pore size) and a linear AB gradient of 0-100% for B over 40 min at a flow rate of 1 mL/min, where eluent A was 0.1% TFA/water and eluent B was 0.1% TFA in 60% CH₃CN/water. Mass was confirmed using MALDI-MS, and Ellman's colour test confirmed the presence of free sulphydryl groups where applicable.

### Production of bicyclic peptides

The crude linear precursors were dissolved in the minimum TFA and diluted to 2 L/0.25 mmol with water. The first disulphide bridge was formed between unprotected Cys residues using K₃Fe(CN)_{6.} The peptide solution was adjusted to pH 7.5 with aqueous ammonium hydroxide. To this solution, 0.01M K₃Fe(CN)₆ was added dropwise to excess, until a slight yellow colour remained. The completion of the reaction was confirmed by HPLC sampling after acidification. The pH of the solution was adjusted to 4 using 50% aqueous acetic acid. The crude reaction mixture was stirred with Bio-Rex 70 weak cation-exchange resin (BioRad, CA) overnight and packed into a glass column. After thorough washing with water, the peptide was eluted using 50% aqueous acetic acid and detected by TLC using ninhydrin. Ninhydrin positive fractions were pooled and lyophilized. Crude material was purified via preparative reverse-phase HPLC. The purified fractions were collected, combined and lyophilised. The second disulphide bridge was formed via I₂-oxidation between Cys(Acm) protected residues. A solution of the peptide (5 mg/ml) in 10% aqueous TFA was mixed vigorously with 8 equivalents of iodine. The course of the reaction was followed using HPLC sampling. At completion of the reaction (usually after 0.5 h), the excess iodine was quenched using 1 M ascorbic acid. The reaction mixture was diluted x 2 with eluent A, filtered through a 0.45µm disposable filter and purified directly via preparative reverse-phase HPLC. The relevant fractions were collected, evaporated, lyophilized and stored at 4°C.

Three peptides of the invention were obtained. They are referred to below as EG 3287 (bicyclic form of SEQ ID NO. 2), EG3307 (bicyclic form of Ac-SEQ ID NO. 2) and EG3315 (bicyclic form of Ac-SEQ ID NO. 2-NH₂).

Full quality control was performed on all purified material. Peptides were shown to be greater than 95% pure by HPLC in two buffer systems (TFA and TEAP). Amino acid analysis (Beckman 6300) following acid hydrolysis confirmed the amino acid composition. MALDI-MS (Kratos Kompact Probe) showed the expected molecular ion. Ellman's colour test was used to confirm the absence of free sulphydryl groups in monocyclic and bicyclic peptides.

### KDR phosphorylation assay

Cells were pretreated with peptide at 10, 30 and 100 mM for 15 minutes followed by treatment with 25ng/ml VEGF for 10 minutes, and cells were immediately extracted by lysis buffer (64 mM Tris-HCl, pH 6.8, 0.2 mM Na₃VO₄, 2% SDS, 10% glycerol, 0.1 mM AEBSF, 5 mg/ml leupeptin). KDR phosphorylation was determined by immunoblotting cell extracts with antibodies which recognise either KDR phosphorylated at Tyrosines 1054 and 1059 (purchased from Oncogene Research Products Inc.) or total KDR (purchased from Santa Cruz Inc.). Immunoreactive bands were visualised by chemiluminescence using horseradish peroxidase-conjugated anti-rabbit IgG and ECL reagent.

Reference may be made to the accompanying drawings. Briefly, Fig. 1 shows effects of cyclised VEGF Exon 7-derived peptides (100 µM) on ¹²⁵I-VEGF₁₆₅ binding to PAE/NP1 cells. In particular, it shows inhibition of VEGF radiolabelled ligand binding to porcine aortic endothelial cells (PAE) expressing only Neuropilin-1 (NP-1) by EG 3287, and no effect of a number of other related cyclic peptides. F9 and F10 refer to two fractions of the same peptide preparation.

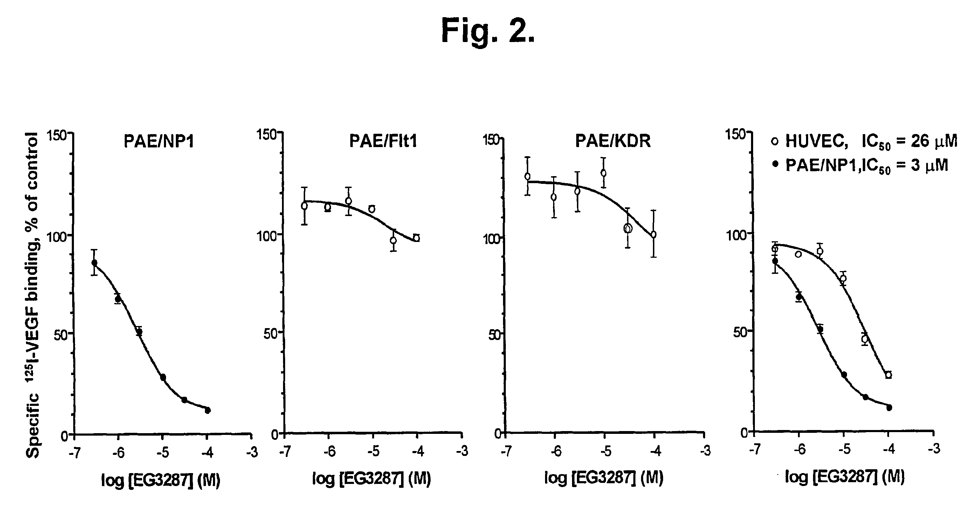

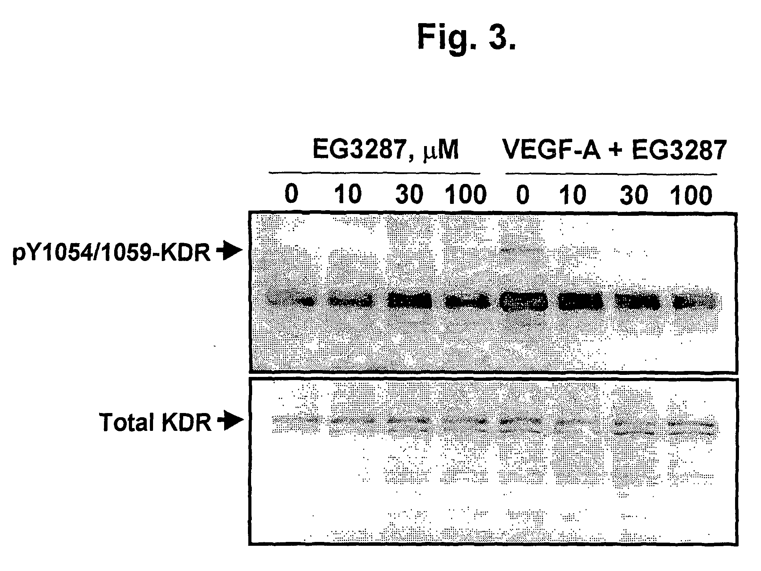

Fig. 2 shows specific, selective inhibition of ¹²⁵I-VEGF₁₆₅ binding to PAE/NP-1 cells, but no effect on binding to cells expressing either only KDR (PAE/KDR) or Flt-1 (PAE/Flt-1), the other two main VEGF receptors. EG3287 also inhibited radiolabelled VEGF binding to human umbilical vein endothelial cells (HUVEC) which express NP-1, KDR and Flt-1 (Fig. 2), and inhibited VEGF-induced KDR phosphorylation (Fig. 3).

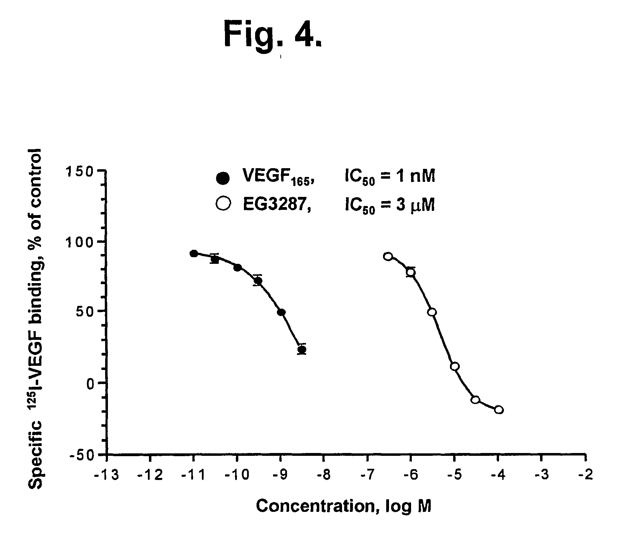

Fig. 4 shows that peptide EG3287 also inhibited binding of radiolabelled VEGF to the breast carcinoma cell line MDA-MB-231, that naturally expresses only NP-1 receptors for VEGF.

## Claims

1. A peptide having the amino acid sequence
SCKNTDSRCKARQLELNERTCRCDKPRR
or a fragment thereof that substantially retains NP-1 antagonist activity, in cyclic form.

2. A peptide according to claim 1, which has the given sequence, in bicyclic form.

3. A peptide according to claim 1 or claim 2, for therapeutic use.

4. Use of a peptide according to claim 1 or claim 2, for the manufacture of a medicament for stimulating nerve repair.

5. Use of a peptide according to claim 1 or claim 2, for the manufacture of a medicament for the treatment of neurodegeneration.

6. Use of a peptide according to claim 1 or claim 2, for the manufacture of a medicament for use in anti-cancer therapy.

## Patentansprüche

1. Peptid mit der Aminosäuresequenz
SCKNTDSRCKARQLELNERTCRCDKPRR
oder ein Fragment desselben, welches im Wesentlichen eine NP-1-Antagonistenaktivität beibehält, in cyclischer Form.

2. Peptid nach Anspruch 1, das die gegebene Sequenz aufweist, in bicyclischer Form.

3. Peptid nach Anspruch 1 oder Anspruch 2 zur therapeutischen Verwendung.

4. Verwendung eines Peptids nach Anspruch 1 oder Anspruch 2 für die Herstellung eines Medikaments zur Stimulierung der Nervenreparatur.

5. Verwendung eines Peptids nach Anspruch 1 oder Anspruch 2 für die Herstellung eines Medikaments zur Behandlung einer Neurodegeneration.

6. Verwendung eines Peptids nach Anspruch 1 oder Anspruch 2 für die Herstellung eines Medikaments zur Verwendung in der Antikrebstherapie.

## Revendications

1. Peptide ayant la séquence d'aminoacides :
SCKNTDSRCKARQLELNERTCRCDKPRR
ou un fragment de celle-ci qui conserve en grande partie une activité antagoniste de NP-1, sous une forme cyclique.

2. Peptide selon la revendication 1, qui possède la séquence donnée, sous une forme bicyclique.

3. Peptide selon la revendication 1 ou la revendication 2, pour une utilisation thérapeutique.

4. Utilisation d'un peptide selon la revendication 1 ou la revendication 2, pour la fabrication d'un médicament destiné à stimuler la réparation de nerfs.

5. Utilisation d'un peptide selon la revendication 1 ou la revendication 2, pour la fabrication d'un médicament destiné au traitement d'une neurodégénérescence.

6. Utilisation d'un peptide selon la revendication 1 ou la revendication 2, pour la fabrication d'un médicament destiné à être utilisé dans une thérapie anti-cancéreuse.
